(19) Europäisches Patentamt
European Patent Office
Office européen des brevets

(11) Publication number: 0 260 606
A2

(12) EUROPEAN PATENT APPLICATION

(21) Application number: 87113263.5

(51) Int. Cl.4: A61K 31/70

(22) Date of filing: 10.09.87

(30) Priority: 11.09.86 JP 214787/86

(43) Date of publication of application:
23.03.88 Bulletin 88/12

(84) Designated Contracting States:
AT BE CH DE ES FR GB GR IT LI LU NL SE

(71) Applicant: MECT CORPORATION
1-1, Nishishinjuku 2-Chome
Shinjuku-ku Tokyo 163(JP)

Applicant: MITSUI TOATSU CHEMICALS, INCORPORATED
2-5, 3-chome, Kasumigaseki
Chiyoda-ku Tokyo(JP)

(72) Inventor: Nagai, Yoshitaka
29-10, Akatsutsumi 1-chome
Setagaya-ku Tokyo(JP)
Inventor: Awaya, Akira
1541, Yabecho Totsuka-ku
Yokohama-shi Kanagawa-ken(JP)
Inventor: Horikomi, Kazutoshi
103, Hagiwara-cho 1-chome
Mobara-shi Chiba-ken(JP)
Inventor: Kobayashi, Hisashi
2141, Togo
Mobara-shi Chiba-ken(JP)
Inventor: Mizuchi, Akira
11-6, Toubudai 3-chome
Mobara-shi Chiba-ken(JP)
Inventor: Shitori, Yoshiyasu
12-6-1307, Nishishinjuku 6-chome
Shinjuku-shi Tokyo(JP)
Inventor: Ito, Masayoshi
27-22-303, Fujimidai 1-chome
Kunitachi-shi Tokyo(JP)
Inventor: Ogawa, Tomoya
6-6-3-101, Kitamachi 3-chome Kichijyouji
Musashino-shi Tokyo(JP)

(74) Representative: Klunker . Schmitt-Nilson . Hirsch
Winzererstrasse 106
D-8000 München 40(DE)

(54) **Medicament for curing neurotic disturbance or disorder comprising sialocyiglycerolipid.**

(57) A medicament composition for curing neurotic disturbance or disorder comprising a compound represented by the following formula (I) of:

EP 0 260 606 A2

Xerox Copy Centre

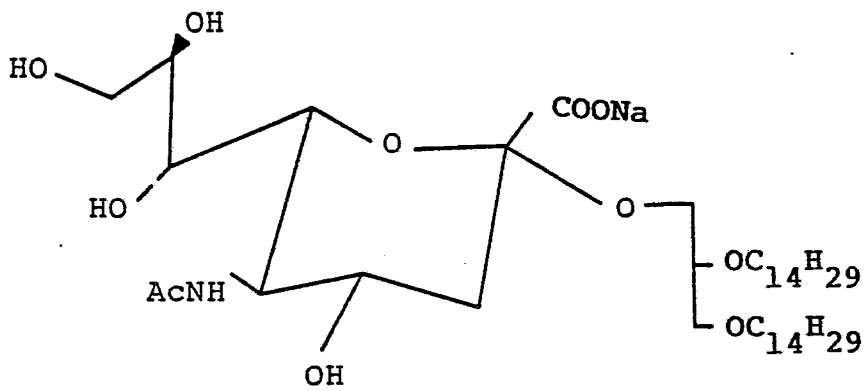

--- (I)

# Medicament for Curing Neurotic Disturbance or Disorder Comprising Sialocylglycerolipid

## BACKGROUND OF THE INVENTION:

### Field of the Invention:

The present invention relates to a lipid derivative containing a sialic acid, which is useful for curing various neurotic diseases caused by disturbance or disorder in peripheral and central nervous systems.

### Description of the Related Art:

In general, the diseases caused by disturbance or disorder in nervous systems are often intractable. However, there are only a few medicaments for curing such diseases at present.

Ganglioside, one of the natural glycolipids, has been used clinically under the Trade Name of Cronassial (see Unexamined Japanese Patent Publication No. 34912/1977: 26323 A/75 filed on August 13, 1975 asserting priority of Italian Application), and Mecobalamine (general name of a group of drugs) have been tried to use for clinical applications. However, the pharmaceutical efficacies of these medicaments did not reach a satisfactory level, and thus there is a demand for a medicament having improved pharmaceutical actions.

Also known in the art as playing an important role in the nervous stimulation conduction systems of animals are acidic glycolipids, such as gangliosides, and acidic phospholipids, such as phosphatidyl inositol and phophatidyl serine. One of the inventors of this invention previously found that ganglyoside mixtures and a certain one of gangliosides acted to promote the proliferation of the first generation cultured nervous cells and neuroblstoma cells and the formation of axis-cylinder process and growth of axis-cylinder process, and that they exhibited effects equivalent to Mecobalamin on the animal models that suffered nervous disorders, and an invention based on this prior finding was the subject of a prior-filed patent application (see Unexamined Japanese Patent Publication No. 222424/1984).

Although the ganglioside mixtures have been tentatively used for practical treatment of peripheral and central nervous system disorders, as has been described above, the gangliosides are natural extracts originating from animals and thus they have the problems of contamination and antigenocity.

Another disadvantage of ganglioside is the difficulty encountered in establishing a strict standard for the preparation of a stable and uniform pharmaceutical composition.

The sialic acid contained in these gangliosides is also contained in glycoproteins and present on the surface of the cells of animals and bacteria; and sialic acid has recently attracted medical and pharmacological interest as a substance which participates in immunoreaction, cancer, inflammation, infection by virus, differentiation of cells and acceptors for hormones. However, to our best knowledge, there has been no report describing a curing effect in the treatment of nervous disorders or diseases by the use of any of the derivatives of lipids containing sialic acid.

Accordingly, the object of this invention is to provide sialocylglycerolipid useful for curing a variety of diseases caused by disturbance or disorder in the peripheral and/or central nervous systems, the sialocylglycerolipid provided by this invention being prepared by modifying the known derivatives of lipids containing sialic acid to convert them into easily soluble alkaline salts, particularly into sodium salts, to be readily applied for use as medicaments.

### Description of the Invention;

The present invention was accomplished on the basis of the finding that the test results on the physiological activities of sialocylglycerolipid conducted while using the neuroblastoma, Neuro2a, have revealed their promotive function on the formation and growth of axis-cylinder process, their activity for repairment and recovery of the nerves of animals suffering from nervous disorders, and their effect for the recovery of motility of animals.

More specifically, the present invention provides a medicament composition for curing neurotic disturbance or disorder characterized in that said composition contains a compound represented by the following formula (I):

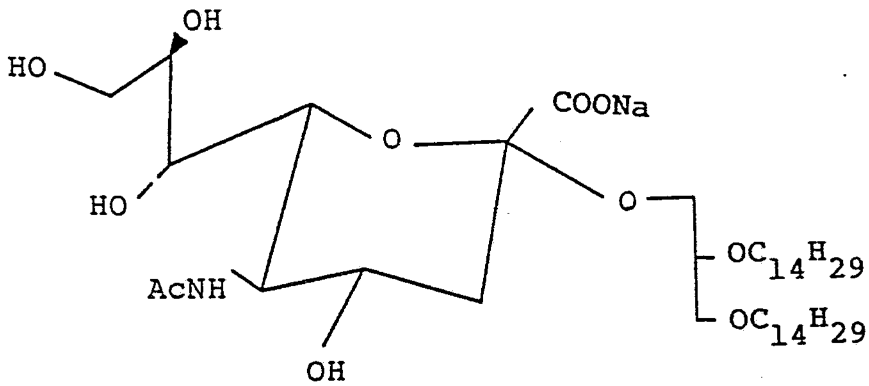

--- (I)

Process for the Preparation of the Compound (I) of the Invention:

The compound (I) may be prepared, for example, by the process as illustrated by the following serial reaction scheme.

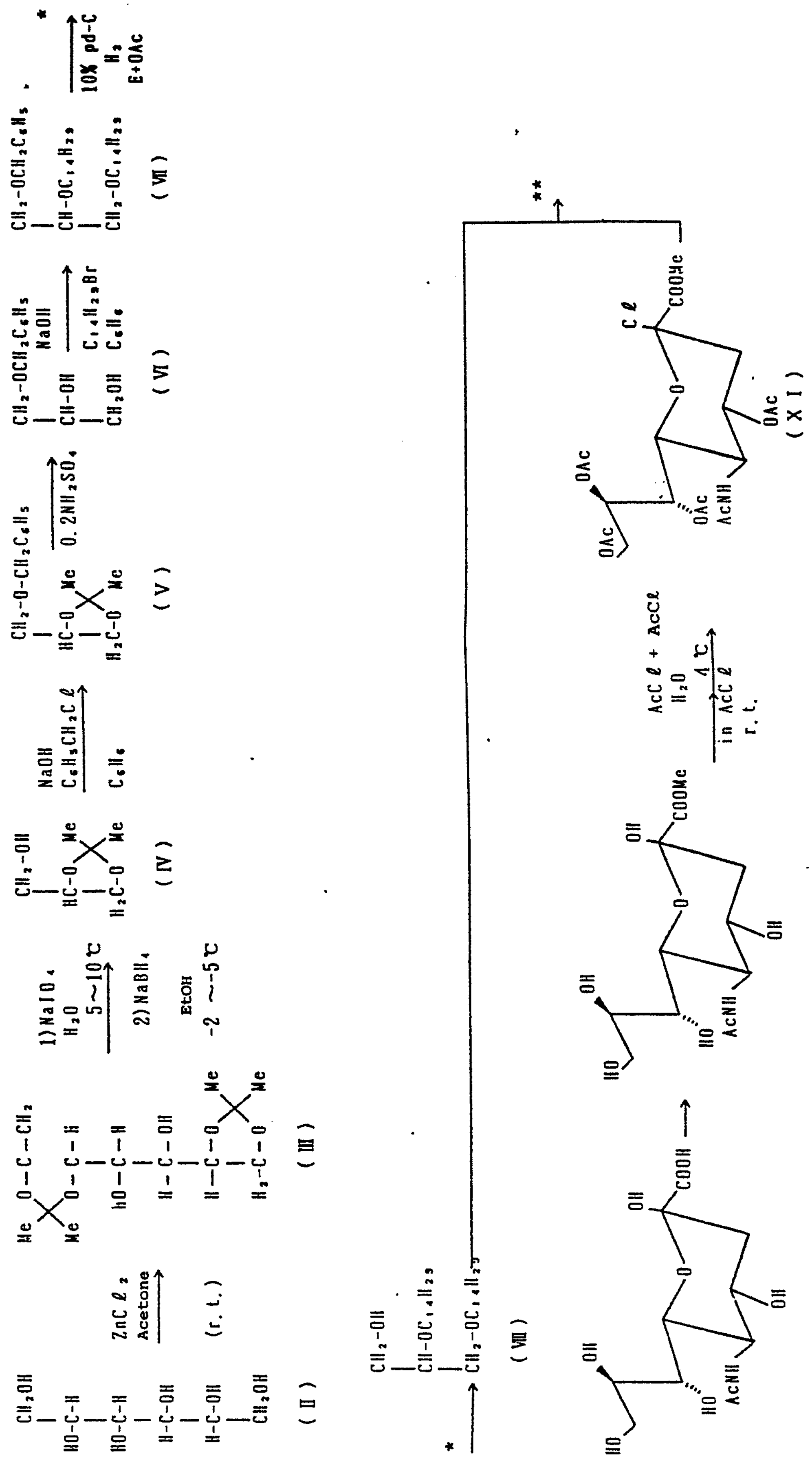


(II) (III) (IV) (V) (VI) (VII) (VIII) (IX) (X) (XI)

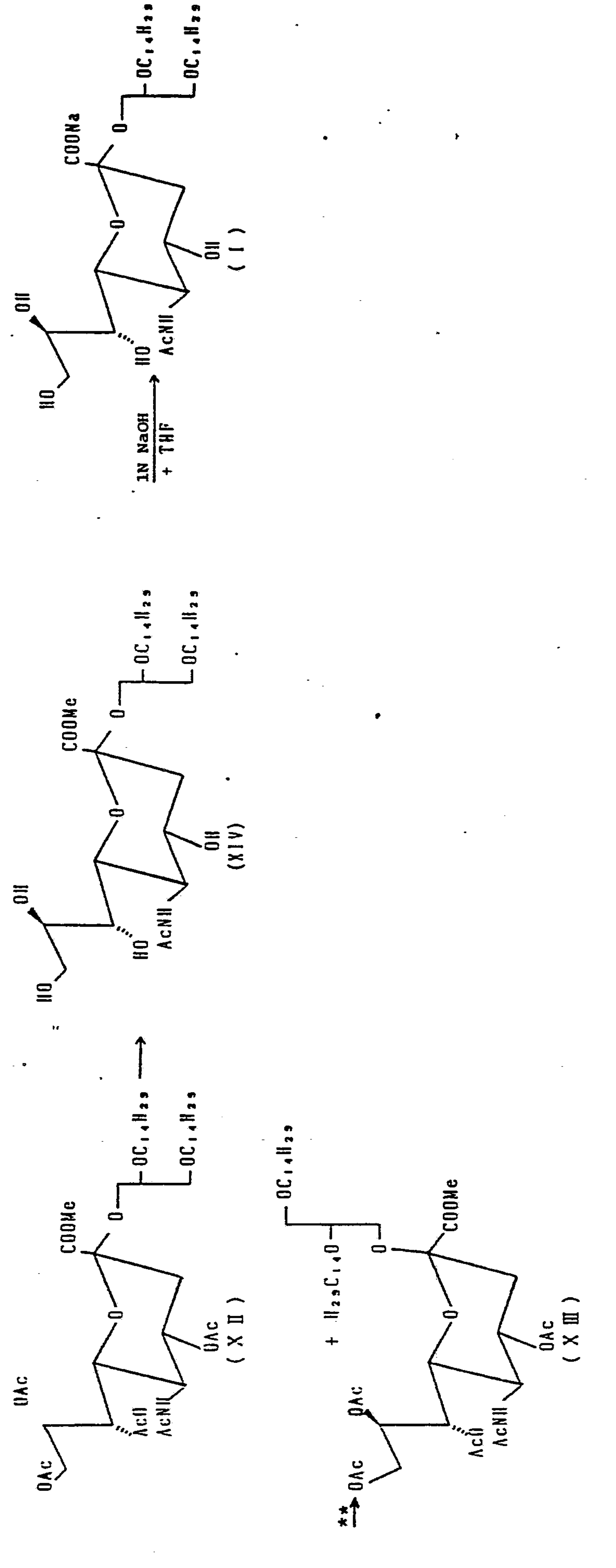


(XII) (XIII) (XIV) (I)

At the initial step, mannitol (II) was used as the starting material and isopropylidenized to produce a compound (III). (See J. Biol. Chem., I28, 463 (I939); J.A.C.S., 67, 338; phytochemistry 2I, 2087 (I982); and Biochem. Prep., 2, 3I (I952). These are incorporated herein as references.)

In the next step, the carbon-carbon bond between the carbon atom of 3-position and the carbon atom of 4-position was ruptured to obtain a compound (IV). (See Phytochemistry, 2I, 3I (I952); and J.A.C.S., 70 609 (I948). These are also be incorporated herein as references.)

The compound (IV) was benzylated to produce a compound (V) (See Biochemistry, 2, 394 (I963; this is incorporated herein as a reference) which was then deisopropylidenized to obtain a compound (VI). (See J.A.C.S., 63, 3244 (I94I); J. Med. Chem., 27, II42 (I984); and Synthesis, 423 (I977). These are incorporated herein as references.) The compound (VI) was reacted with I-bromotetradecane, followed by debenzylation, to produce a compound (VIII). (See Agric. Biol. Chem., 46(I), 255 (I982); and Biochemistry 2, 394 (I963). These are incorporated herein as references.)

Separately, in accordance with the method of Kuhn et al. (see Chem. Ber., 99, 6II (I966); this is incorporated herein as a reference, a compound (XI) was prepared from sialic acid (N-acetylneuraminic acid; compound (IX)) through a compound (X).

The compound (XI) was reacted with the compound (VIII) to produce mixture of a compound (XII) (α-isomer) and a compound (XIII) (β-isomer), from which the compound (XII) was isolated and then deacetylated to obtain a compound (XIV).

Method of Using the Compound (I) of the Invention:

The compound (I) of the invention may be used in ordinary dosage forms conventionally used for administering a variety of pharmaceutical compositions. Any of the known administration forms may be adopted, including injection forms, such as intramuscular injection, intravenous injection and subcutaneous injection, eye-dropping lotion, inhalant and transmucous administration form. The compound (I) of the invention may also be mixed with a variety of pharmaceutically acceptable carriers to prepare various type preparations including the aforementioned injection forms and eye-dropping lotion, and other type preparation forms, such as cataplasm and suppository.

The compound of this invention may be contained in any desired preparation forms so that it is released from the preparations rapidly, continuously or in a delayed mode.

When the compound of the invention is to be administered non-orally, the active component, i.e. one or more of the compounds (I), may be dissolved in a I0% aqueous glucose solution, isotonic saline solution or distilled water for an injection, and may be administered through intramuscular injection or be sealed in a vial ampule to be ready for administration through intravenous drip or injection. The solutions may also be freeze-dried after they have been contained in vial ampul, in order to ensure more stable storage thereof.

The quantity of the compound (I) of the invention to be adminstered to a patient varies depending on the degree of the disease and the weight of the patient, and it is preferred that 0.I to 5 mg of the active components be contained in a single dose.

The preparation examples and the biological activities of the compound of this invention will now be described in detail while referring to some Examples and Preparation Examples. However, it should be noted here that the present invention is not limited only to the following Examples and Preparation Examples.

Preparation Example I: Preparation of Injection

An injection was prepared by putting 0.5 mg of the compound (I), 0.25 mg of sodium dihydrogenphosphate dihydrate, 3 mg of disodium hydrogenphosphate dodecahydrate into an ampul and added with distilled water to adjust the volume in the ampul to I ml.

Preparation Example 2: Preparation of Injection Which is Dissolved Prior to Use.

Into a vial put were 0.5 mg of the compound (I) and I ml of physiological saline solution, and the content of the vial was freeze-dried. In use, the thus prepared freeze-dried product was dissolved in I ml of distilled water for the preparation of an injection.

Preparation Example 3: Preparation of Eye-dropping Lotion

An eye-dropping lotion was prepared by putting I mg of the compound (I), 52.5 mg of boric acid, I4.5 mg of borax and 0.I5 mg of benzalkonium chloride into a vial, and added with a dissolution medium for the dropping lotion so that the total volume in the vial was adjusted to 5 ml.

Preparation Example 4: Preparation of Inhalant

The compound (I) was put into an agate mortar and ground sufficiently to prepare a fine powder having a particle size of I to 20 microns. The powdered compound (I) was admixed with lactose, and the admixture was pulverized again. The finely pulverized admixture was added with additional lactose little by little, while grounding and mixing throughly, to prepare a powder mixed with 20 to 40 times volume of lactose. Capsules and containers (folded powder) were each charged with 20 to 40 mg of the powder. The content of the capsules was used as a powder-form aerosol inhalant, and the content of the folded powder was used as a liquid-form aerosol inhalant.

Biological Activity of the Compound (I) of the Invention:

The biological activity of the compound (I) on the nervous cell was tested in vitro. The nervous cell used in the test was neuroblastoma cell Neuro2a which is readily available and can be cultured in a relatively simple manner. The cell was cultured in a serum-containing culturing solution and then transferred to a serum-free culturing solution to which the compound (I) was aseptically added, and the change of the cultured cell after the lapse of a predetermined time was observed.

The compound (I) was found to have a significant effect of promoting the growth of the axis-cylinder process, as compared with the control group.

The in vivo effects of the compound (I) on rats of mice were tested in the following manner. The nigral dopaminergic neuron of the brain of a rat was chemically destroyed by injecting a very small quantity of 6-hydroxydopamine to cause motor disturbance.

After the lapse of two weeks, a fetal brain dopaminergic cell was transplanted on the Nucleus caudatus of the rat brain to try to remedy the motor disturbance. More specifically, the compound (I) was administered every day for two weeks from the day on which the dopamin ergic cell was transplanted, and the effects of the compound (I) for the remedy of the motor disturbance and on the growth of the transplanted cell were examined. It was found that the compound (I) has an effect of promoting the remedy of motor disturbance similar to the ganglioside mixtures.

It was also found that the compound (I) promoted the recovery to the normal values of the distance between the toes and the weight of the musculi soleus in a rat whose sciatic nerve was crushed.

It was thus found that the compound (I) of the invention had utility when used as a curing agent or medicament for remedying the nervous diseases, such as disturbances in peripheral or central nervous systems, of mammals.

Typical of such diseases of the nervous system are a variety of neuropathies, for example, various peripheral nervous disorders accompanied with motorgenetic, perceptive or objective reflex retardation including diseases in nervous root due to traumatic, inflammatory and immunological causes, peripheral nervous disorders caused by alcohol or drugs, metabolic or idophathic disorders in peripheral nervous system caused by, for example, glucosuria. More particularly, such diseases include facial palsy, paralysis of the sciatic nerve, spinal muscular atorophy, muscular dystrophy, serious myasthenia, multiple sclerosis, muscular artrophic Seitenstrangsklerose, acute scattering Encephalitis or Myelitis, Guillain-barré syndrome, Encephalitis after the inoculation of vaccine, SMON disease, dementia, Altzheimer's disease syndrome, recuperation from damage on the base of the skull, ischemia, after-effect of cerebral infarction or ventricular hemorrhage and rheumatism. The diseases set forth above are illustrative only, and the compound of this invention may be used for the treatment of other diseases.

Toxicity Test of the Compound (I):

The result of the toxicity test of the compound of this invention revealed that it had extremely low toxicity and thus may be used as a safe drug. (In this connection, reference should be made to Example 3.)

Example I: Effect on the Neuroblastoma Cell, Neuro2a

Neuro2a was floated on a culture medium containing fetal cattle serum (hereinafter referred to as FCS), the culture medium consisting of 90% of Dubecco's Modified Eagle's Medium (hereinafter referred to as MEM) and I0% of FCS and added with I00 U/ml of penicillin G and I00 µg/ml of streptomycin sulfate, and cultured in a carbon dioxide cultivator at 37°C in an air containing 5% of carbon dioxide. The containers used were 60 mm polystyrene dishes, and each of the containers was incubated with I to 2 $\times$ I0$^4$ cells followed by culturing for 48 hours prior to the test. After removing the culture medium containing FCS, respective cell culturing dishes were added with a culture medium which did not contain PSC (MEM: I00%, added with the same concentrations of the aforementioned antibiotics) and admixed with a test sample selected from the compound (I), Gal, (βI-3) GalNAc (βI-4) NAcNeu(α2-3))Gal(βI-4)Glc(βI-I)Ceramide (hereinafter referred to as $GM_1$) and (NAcNeu(α2-8) NAcNeu(α2-3))-Gal(βI-3)GalNAc(βI-4)(NAcNeu(α2-8)-NAcNeu(α-2-3))-Gal(βI-4)Glc(βI-I)(hereinafter referred to as $GQ_{1b}$). The cultivation of cells was continued after the addition of FCS-free culture media each containing a predetermined quantity of the compound (I), $GM_1$ or $GQ_{1b}$. After the lapse of 24 hours and 48 hours, the increase in cells formed with axis-cylinder process, the increase in number of axis-cylinder processes and the elongation of the length of the axis-cylinder process were measured. The results are shown in Tables I to 3.

The test was conducted while using three dishes for each concentration of each test material.

The results are shown by the average value ± standard error (S.E.).

## Table 1

After the lapse of 24 hours

| Material Tested (μg/ml) | Total Number of Cells | Number of Cells Formed with Axis-cylinder Process | Ratio of Cells Formed with Axis-cylinder Process (%) | Length of Axis-cylinder Process (μm/cell) | Number of Axis-cylinder Process (per cell) |
|---|---|---|---|---|---|
| 0 | 434 | 200 | 46.1 | 117.9 ± 4.93 | 1.3 ± 0.04 |
| Compound (I) 0.01 | 412 | 184 | 44.7 | 107.5 ± 4.56 | 1.2 ± 0.03 |
| Compound (I) 0.1 | 369 | 188 | 50.9 | 111.4 ± 4.60 | 1.2 ± 0.03 |
| Compound (I) 1 | 337 | 214 | 63.5 | 158.7 ± 6.94* | 1.0 ± 0.02 |
| Compound (I) 10 | U.C. | U.C. | ---- | U.C. | U.C. |
| After the lapse of 48 hours (μg/ml) | | | | | |
| 0 | 589 | 264 | 44.8 | 95.2 ± 3.54 | 1.1 ± 0.03 |
| Compound (I) 0.01 | 491 | 249 | 50.7 | 117.1 ± 4.94* | 1.2 ± 0.03 |
| Compound (I) 0.1 | 464 | 258 | 55.6 | 128.8 ± 5.71* | 1.2 ± 0.03 |
| Compound (I) 1 | 404 | 248 | 61.4 | 154.7 ± 5.99* | 1.1 ± 0.03 |
| Compound (I) 10 | U.C. | U.C. | ---- | U.C. | 1 U.C. |

Note: U.C. = uncountable, * $p < 0.001$

Table 2

After the lapse of 24 hours

| Material Tested (µg/ml) | | Total Number of Cells | Number of Cells Formed with Axis-cylinder Process | Ratio of Cells Formed with Axis-cylinder Process (%) | Length of Axis-cylinder Process (µm/cell) | Number of Axis-cylinder Process (per cell) |
|---|---|---|---|---|---|---|
| | 0 | 320 | 146 | 45.6 | 101.0 ± 6.04 | 1.3 ± 0.05 |
| $GM_1$ | 0.01 | 334 | 145 | 43.4 | 94.9 ± 6.64 | 1.4 ± 0.05 |
| $GM_1$ | 0.1 | 297 | 142 | 47.8 | 101.1 ± 5.09 | 1.4 ± 0.06 |
| $GM_1$ | 1 | 332 | 140 | 42.2 | 109.7 ± 6.44 | 1.4 ± 0.06 |
| $GM_1$ | 10 | 325 | 149 | 45.8 | 89.3± 4.77 | 1.4 ± 0.05 |
| $GM_1$ | 100 | 310 | 172 | 55.5 | 90.5 ± 3.88 | 1.8 ± 0.05 |
| After the lapse of 48 hours (µg/ml) | | | | | | |
| | 0 | 383 | 184 | 48.0 | 95.8 ± 4.61 | 1.4 ± 0.05 |
| $GM_1$ | 0.01 | 333 | 194 | 58.3 | 93.7 ± 5.07 | 1.4 ± 0.05 |
| $GM_1$ | 0.1 | 349 | 156 | 44.7 | 108.8 ± 5.97 | 1.4 ± 0.06 |
| $GM_1$ | 1 | 352 | 211 | 59.9 | 109.2 ± 6.09 | 1.3 ± 0.04 |
| $GM_1$ | 10 | 330 | 205 | 62.1 | 113.8 ± 5.49** | 1.3 ± 0.04 |
| $GM_1$ | 100 | 317 | 193 | 60.9 | 98.9 ± 5.22 | 1.5 ± 0.05 |

Note: *** $p < 0.05$

Table 3

After the lapse of 24 hours

| Material Tested (μg/ml) | | Total Number of Cells | Number of Cells Formed with Axis-cylinder Process | Ratio of Cells Formed with Axis-cylinder Process (%) | Length of Axis-cylinder Process (μm/cell) | Number of Axis-cylinder Process (per cell) |
|---|---|---|---|---|---|---|
| | 0 | 320 | 146 | 45.6 | 101.0 ± 6.04 | 1.3 ± 0.05 |
| $GQ_{1b}$ | 0.01 | 336 | 174 | 51.8 | 88.5 ± 5.00 | 1.3 ± 0.05 |
| $GQ_{1b}$ | 0.1 | 331 | 159 | 58.0 | 86.0 ± 4.72 | 1.3 ± 0.04 |
| $GQ_{1b}$ | 1 | 308 | 157 | 51.0 | 97.6 ± 4.95 | 1.4 ± 0.05 |
| $GQ_{1b}$ | 10 | 321 | 205 | 63.9 | 114.9 ± 5.98 | 1.3 ± 0.06 |
| $GQ_{1b}$ | 100 | U.C. | U.C. | ---- | U.C. | U.C. |
| After the lapse of 48 hours (μg/ml) | | | | | | |
| | 0 | 383 | 184 | 48.0 | 95.8 ± 4.61 | 1.4 ± 0.05 |
| $GQ_{1b}$ | 0.01 | 371 | 158 | 42.6 | 100.4 ± 5.76 | 1.5 ± 0.06 |
| $GQ_{1b}$ | 0.1 | 347 | 153 | 44.1 | 90.4 ± 6.06 | 1.2 ± 0.04 |
| $GQ_{1b}$ | 1 | 384 | 182 | 47.4 | 106.5 ± 6.40 | 1.3 ± 0.04 |
| $GQ_{1b}$ | 10 | 346 | 214 | 61.8 | 122.5 ± 6.99** | 1.3 ± 0.04 |
| $GQ_{1b}$ | 100 | U.C. | U.C. | ---- | U.C. | U.C. |

Note: U.C. = Uncountable, ** $P < 0.01$

Result :

The minimal effective concentration of the compound (I), at the time point of 48 hour cultivation, was compared with those of $GM_1$ and $GQ_{1b}$ to find that the minimal effective concentration of the compound (I) was 0.0l µg/ml while that of $GM_1$ was also l0µg/ml and that of $GQ_{1b}$ was also l0 µg/ml. In consideration of the molecular weights of these compounds, the compound (I) exhibited activity 500 times as high as that of $GM_1$ and 300 times as high as that of $GQ_{1b}$. The result showed that the compound (I) had an extremely high activity for promoting growth or elongation of axis-cylinder process of the nerve.

Example 2: Remedying and Remedy Promoting Effects Obtainable by the Transplantation of Fetal Cerebral Cell for Curing Motor Disturbance Caused by Damage in Rat Brain Cell

The nigral depaminergic neuron at the left side of the brain of a female Wister family rat aged 4-weeks (Body Weight: l00 g) was destroyed by injecting a very small quantity of 6-hydroxydopamine. The rat showed a tendency to spontaneously rotate in the direction opposite to the destroyed side for several days, but not no apparent abnormal action was observed after that. Upon administration of methamphethamine (5 mg/kg, i.p.) to the rat having its nigral depaminergic neuron destroyed, it began rotational movement in the direction toward the destroyed side.

After two weeks from the destruction by the administration of the drug, portions of the Truncus corporis callosi containing dopamin cells (i.e. substantia nigra and the Tegmentum at the abdomen side) were extracted from the fetal rat brain of a fetal rat of l4 to l7 days age, cut finely, and treated with trypsin. Then, the extracted tissues were incubated at 37°C for 30 minutes, and the tissues were subjected to pipetting to form a suspension. 5 µl each of the suspensions were transplanted on two sites of the Nucleus caudatus of the destructed side, the total of l0 µl (containing about $l0^5$ cells) being transplanted.

30 mg/kg (i.P.) of the compound (I) was administered every day for two weeks from the day of transplantation. The rotational movements caused by administration of methamphethamine were examined at the time point before 2 weeks of the transplantation (-2W), at the time point before one week of the transplantation (-lW) and at the time point after two weeks of the transplantation (2W). The number of rotational movements within one minute were counted at l0 minute intervals after six administrations of methamphethamine, and the total number of rotational movements counted for the six times was averaged to find the mean rotational movement. As controls, the numbers of rotational movements of the rats administered, respectively, with a ganglioside mixture and $GM_1$ ganglioside were counted. The results are shown collectively in Table 4.

Table 4: Effect of Compound (I) Affecting on the Rotational Movement of Rats Induced by Methamphethamine

| | Number and Average of Rotational Movement of Rats (Average + S.D.) Lapse of Week after the Transplantation of Melanodopamin Cell | | |
|---|---|---|---|
| | - 2W | - 1W | 2W |
| Experiment 1 | 12.2 | 17.4 | - 3.0 |
| Compound (I) of | 5.8 | 12.2 | 5.3 |
| the Invention | 5.4 | 8.4 | 3.2 |
| 30 mg/kg | 9.5 | 11.0 | 5.8 |
| | 8.2+3.2 | 12.3+3.8 | 2.8+4.0* |
| Pysiological | 9.7 | 10.0 | 9.7 |
| Saline Solution | 6.0 | 7.7 | 3.3 |
| | 9.8 | 9.2 | 12.3 |
| | 17.0 | 19.0 | 4.7 |
| | 10.6+4.6 | 11.5+5.1 | 7.5+4.2 |
| Experiment 2 | 12.5 | 14.8 | 3.7 |
| Ganglioside $GM_1$ | 10.1 | 6.9 | 10.9 |
| 30 mg/kg | 4.5 | 7.6 | 8.2 |
| | 9.0+4.1 | 9.8+4.4 | 7.6+3.6 |
| Ganglioside Mixture | 6.4 | 8.8 | 3.7 |
| 50 mg/kg | 11.6 | 11.0 | 4.0 |
| | 8.3 | 7.5 | 6.0 |
| | 8.8+2.6 | 9.1+1.8 | 5.6+1.4** |
| Physiological | 6.5 | 8.6 | 9.0 |
| Saline Solution | 5.5 | 12.0 | 9.0 |
| | 13.9 | 7.8 | 10.3 |
| | 8.6+4.6 | 9.5+2.2 | 9.4+0.7 |

Comparison between the Groups Administered with Drugs and the Group administered with Raw Feed:

Student's T-test * $0.05 < P < 0.1$, ** $0.01 < P < 0.05$

The numbers of the rotational movements of the rats administered with the drugs were significantly decreased as compared with those of the rat administered with the physiological saline solution; and it was clearly seen that the compound (I) had a function of remedying the nerves similar to gangliosides used as the control drugs.

Example 3: Effect of Curing Rat Having its Sciatic Nerve Crushed.

The curing effect of the compound (I) of the invention on the rats having their sciatic nerves crushed, which were used as models caused by peripheral nervous disturbances, was assessed by examining (I) the change in action of the crushed hind leg and (2) the change in weight of the muscle as indicators for the modification of peripheral nerve and for the recovery course of the disturbance.

In the experiment, seven male Wister family rats (6 weeks age) were used per a group. The sciatic nerve of each rat was crushed in accordance with the method of Yamazu et al. (see Kiyomi Yamazu, Takenori Kaneko, Akifumi Kitahara and Isao Ohkawa, Journal of Japanese Pharmacological Society, 72, 259 - 268 (l976)) and in accordance with the method of Hasegawa et al. (see Kažuo Hasegawa, Naoji Mikuni and Yutaka Sakai, Journal of Japanese Pharmacological Society, 74, 72l - 734 (l978)). Specifically, under anesthesia with Pentobarbital (40 mg/kg, i.p.), the left sciatic nerve was exposed at the femur and the site 5 mm centrally from the branch portion between the N. tibialis and the N. suralis was crushed using a modified artery klemme having a width of 2 mm and gap of 0.l mm. Rats subjected to the operation were randomly dispensed to form the groups subjected to test.

The compound (I) of the invention was intraperitoneally administered one time per day from the day on which the nerve was crushed to the 22nd day. The control groups included a group administered with Mecobalamin (produced by Gedeon Richter Ltd.), a group administered with a 20% aqueous solution of propylene glycol (hereinafter referred to as 20% PG) which was used to dissolve the compound (I) of the invention, and a group administered with a 0.9% aqueous saline solution. The predetermined measurements were conducted with the lapse of time after the crushing operation (at the first, fourth, seventh, tenth, l4th, l5th, l7th, l9th 2lst and 28th days).

(I) Change in Action of the Crushed Hind Leg:

The distance between the toes was measured, since this is a good indicator showing the modification and recovery of the function of the nerve and the change thereof could conveniently measured with the lapse of time.

The measurement was conducted in accordance with the method of Hasegawa, (Hasegawa, K., Experientia, 34 , 750 - 75l (l978)) by measuring the distance from the first toe to the fifth toe of the hind leg.

The ratio of the distance at the crushed side to the distance at the normal side was calculated as a percentage (%), the average values and the standard deviations (S.D.) being shown in Table 5.

The values of the test groups which are significantly different, by the t-test of Student, from that of the control group administered with an aqueous saline solution are afixed at the upper right with suffix * for those where $p<0.05$ and with a suffix ** for those where $p<0.0l$.

The distance between the toes at the crushed side immediately after the crushing operation was about a half (50%) of the distance at the normal side, the ratio declining until the tenth day, and no significant difference was found between the groups. However, after the l5th day, the groups administered with the drugs showed rapid recoveries resulting in significant differences from the control groups administered with the 20% PG and the raw feed. At the 20th to 2lst day, the control groups recovered to a level approximate to (about 90% of) the groups administered with the drugs.

(2) Change in Weight of Muscle:

It is known that removal of or disturbance in a certain nerve causes atrophy of the muscle controlled by the nerve, and the atrophy is gradually recovered by the recontrol by the nerve. For this reason, the quantitative change in weight of the muscle was selected as an indicator. At 28th day after the operation, the muscli soleus of both hind legs were extracted under anesthesia with Pentobarbital, and the weights of the muscles were weighed. The weight ratio of the muscli soleus at the crushed side to the muscli soleus at the normal side was calculated and represented by percentage (%). The average values and the standard deviations of respective groups are shown in Table 5.

Table 5: Curing Effect on Rats Having Their Sciatic Nerves Crushed

| Material Dosed | Dosage | Recovery Rate (%) in Distance between the Fingers | | Recovery Rate (%) in Weight of the Muscle |
|---|---|---|---|---|
| | | 17th Day | 19th Day | 28th Day |
| 20% PG | 1 ml/kg | 66.3 ± 5.4 | 82.2 ± 6.5 | 83.2 ± 8.2 |
| Compound (I) of the invention | 3 mg/kg, ip | 71.5 ± 5.5* | 82.1 ± 9.7 | 82.8 ± 6.7 |
| | 30 mg/kg, ip | 80.4 ± 9.6** | 90.3 ± 4.4** | 89.1 ± 5.7 |
| Saline | 1 ml/kg | 69.7 ± 7.0 | 81.8 ± 5.2 | 82.4 ± 7.5 |
| Mecobalamine | 0.5 mg/kg, ip | 77.0 ± 10.7 | 88.8 ± 5.1** | 82.7 ± 5.1 |

Compared respectively with the group dosed with the 20% PG and the group dosed with the raw feed through the Student's T-test: * $P < 0.05$, ** $P < 0.01$

Ten rats were used in each group.

Example 4:Effect on Remedy and Restoration of Learning and Memory of Mice Having Their Nerves Damaged by Mercurialism

Male Balb/c mice of 7-weeks age were pre-trained in a T-shaped labylinth 3 times a week so that they learned to go straight from the starting area to the safety area. Then, a group of mice was orally administered methylmercuric chloride (hereinafter referred to as MMC) at a dose of 6 mg/kg/day for 7 days after the mice had reached 8-weeks age. A control group of mice was administered 0.l ml/l0 g/day of saline. The day after the completion of administration of MMC, the compound (I) of the invention was intraperitoneally administered at a dose of 40 mg/kg/day for l0 days. From the 23rd day, the training in the T-shape labylinth was restarted, and the running actions of the mice were observed. At the 7th day (29th day from the beginning of the experiment) and 8th day (30th day from the beginning of the experiment) from the re-starting of the training, the number of mice of each group, that could be subjected to test, was counted; and the number of mice that reached the standard (running into the safety area within 5 seconds) for 8 or more times per ten times trials was counted. In the following Table 6, the number of mice subjected to test is shown as the denominator, and the number of mice that reached the standard is shown as the numerator. Meanwhile, the decrease in number of mice was caused by death due to poisoning from MMC. Also shown in Table 6 are the average times (second) ± S.D. (standard error) required for running into the safety area. The results revealed that the compound (I) of the invention had the effect of remedying and restoring the learning and memory ability mice.

Table 6: Effect on Remedy and Restoration of Learning and Memory Ability of Mice Having Their Nerves Damaged

| Treatment | 7th Day | 8th Day |
|---|---|---|
| Saline, 0.1 ml/10g/day | 7/7 3.3±1.3 sec. | 7/7 2.9±0.6 sec. |
| MMC | 0/3 - | 1/3 - |
| MMC + Compound (I) 40 mg/kg,i.p./day | 4/6 2.5±0.8 sec. | 5/6 3.1±0.7 sec. |

Note: "-" means that there is no average value.

Example 5: Acute Toxicity Test

The compound (I) was administered through two administration routes, i.e. orally and intravenous injection, to the groups each consisting of five male ddy mice, while increasing the dosage amounts little by little.

From the time immediately after the administration to the lapse of 7 days, the general syndrome and death of the mice were inspected. The medial lethal dose ($LD_{50}$) was calculated, through the Probit method, from the mortality rate at the 7th day from the beginning of administration.

$LD_{50}$ = 58l mg/kg (through intraveous injection)

5000 mg/kg (through oral administration)

As will be appreciated from the foregoing, the compounds (I) of the invention may be conveniently used as a medicament for curing neurotic diseases including the disturbances or disorders in the peripheral and central nervous systems, since they exhibit promotive function on the neurite outgrowth when applied to a nervous cell (neuroblasto tumor cell, Neuro2a) and also exhibit an effect of restoring the nerves and an effect of restoring the motor function in rats having their nerves damaged.

As described in Examples I and 2, the compounds (I) have the biological activities similar to those of gangliosides which have been used as the control materials in the Examples.

The toxicity of the compounds (I) of the invention is low, as shown by Example 3. Accordingly, the compounds (I) can be used as efficacious medicaments which have high activities, toxicity and high safety factor.

## Claims

I. A medicament composition for curing neurotic disturbance or disorder characterized in that said composition contains a compound represented by the following formula (I) of:

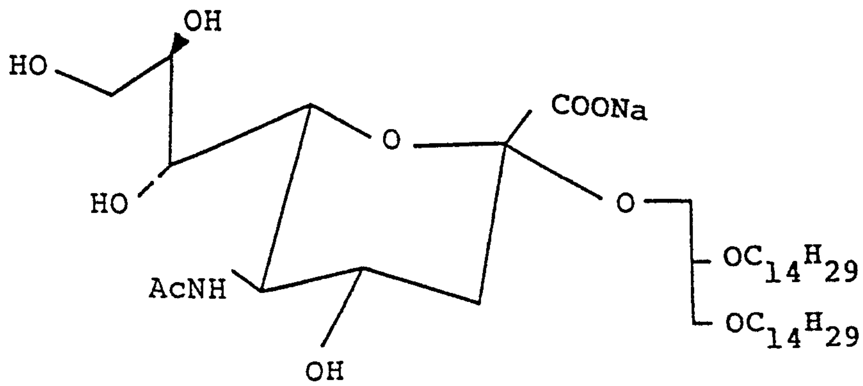

--- (I)

2. The medicament composition for curing neurotic disturbance or disorder according to claim I, wherein said composition is in the form of an eyedrop lotion.

3. The medicament composition for curing neurotic disturbance or disorder according to claim I, wherein said composition is in the form of an inhalant.

4. The medicament composition for curing neurotic disturbance or disorder according to claim I, wherein said composition is in the form of an injection.

5. The medicament composition for curing neurotic disturbance or disorder according to any one of claims 1 to 4, wherein said composition is in the form of a preparation for dosage containing 0.1 to 5 mg of pharmacologically active component.